(19) **Europäisches Patentamt / European Patent Office / Office européen des brevets**

(11) **EP 3 263 525 B1**

(12) **EUROPEAN PATENT SPECIFICATION**

(45) Date of publication and mention
of the grant of the patent:
**31.03.2021 Bulletin 2021/13**

(21) Application number: **16755541.6**

(22) Date of filing: **24.02.2016**

(51) Int Cl.:
*C01G 9/02* (2006.01)     *A61K 8/27* (2006.01)
*A61K 8/81* (2006.01)     *C01G 9/00* (2006.01)
*A61K 8/25* (2006.01)     *A61Q 17/04* (2006.01)

(86) International application number:
**PCT/JP2016/055405**

(87) International publication number:
**WO 2016/136797 (01.09.2016 Gazette 2016/35)**

(54) **SILICON OXIDE-COATED ZINC OXIDE, SILICON OXIDE-COATED ZINC OXIDE-CONTAINING COMPOSITION, AND COSMETIC PRODUCT**

SILICIUMOXIDBESCHICHTETES ZINKOXID, ZUSAMMENSETZUNG MIT SILICIUMOXIDBESCHICHTETEM ZINKOXID UND KOSMETISCHES PRODUKT

OXYDE DE ZINC RECOUVERT D'OXYDE DE SILICIUM, COMPOSITION CONTENANT DE L'OXYDE DE ZINC RECOUVERT D'OXYDE DE SILICIUM ET PRODUIT COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2015 JP 2015038674**

(43) Date of publication of application:
**03.01.2018 Bulletin 2018/01**

(73) Proprietor: **Sumitomo Osaka Cement Co., Ltd.**
**Tokyo 102-8465 (JP)**

(72) Inventors:
• **SUMA Syunsuke**
 **Tokyo 102-8465 (JP)**
• **ITAGAKI Tetsuro**
 **Tokyo 102-8465 (JP)**
• **FUJIHASHI Gaku**
 **Tokyo 102-8465 (JP)**
• **MATSUSHITA Hirokazu**
 **Tokyo 102-8465 (JP)**

(74) Representative: **Cabinet Laurent & Charras**
**Le Contemporain**
**50 Chemin de la Bruyère**
**69574 Dardilly Cedex (FR)**

(56) References cited:
**WO-A1-2014/171322     WO-A1-2015/072499**
**WO-A1-2015/072499     JP-A- H10 130 021**
**JP-A- H11 302 015     JP-A- 2002 087 817**
**JP-A- 2009 280 547     JP-A- 2011 102 291**
**JP-A- 2013 006 375**

• **DATABASE WPI Week 200260 Thomson Scientific, London, GB; AN 2002-560457 XP002782285, & JP 2002 087817 A (SUMITOMO CEMENT CO LTD) 27 March 2002 (2002-03-27)**
• **DATABASE WPI Week 200003 Thomson Scientific, London, GB; AN 2000-033404 XP002782286, & JP H11 302015 A (SAKAI KAGAKU KOGYO KK) 2 November 1999 (1999-11-02)**

**Description**

Technical Field

The present invention is according to the claims and relates to silicon oxide-coated zinc oxide, a composition containing silicon oxide-coated zinc oxide, and a cosmetic product.

The present application claims a priority based on Japanese Patent Application No. 2015-038674 filed on February 27, 2015,

Background Art

The cosmetic products on which an ultraviolet shielding function is conferred are frequently used not only for leisure activities but also for daily use. Therefore, it is important for the cosmetic products to have a texture which does not cause stress such that they can be used every day. In order to obtain such a texture, there is a demand for water-based cosmetic products having a fresh texture.

The water-based cosmetic products are not sticky unlike oil-based cosmetic products and have a dry texture. Therefore, in recent years, the water-based cosmetic products have been used as various cosmetic products such as a sunscreen agent, an emulsion, and a cream. In these cosmetics, titanium oxide and zinc oxide are mainly used as inorganic ultraviolet shielding agents.

Incidentally, zinc oxide has photocatalytic activity. Therefore, in a case where zinc oxide is used in cosmetic products, there are inconveniences in that the mixing amount of zinc oxide or other coexisting mixing components should be carefully selected. Accordingly, in order to suppress the photocatalytic activity of zinc oxide, surface-coated zinc oxide is suggested which is obtained by coating the surface of zinc oxide with a substance having low activity such as silicon oxide or zirconium oxide (for example, see Patent Literature Nos. 1 and 2).

However, in a case where zinc oxide is used in water-based cosmetic products, the problems specific to the water-based cosmetic products occur. That is, because of being an amphoteric metal, zinc oxide easily dissolves in an acid or alkali or even dissolves in water in a trace amount, and unfortunately, zinc ions are released. Accordingly, in a case where zinc oxide is used in water-based cosmetic products, the eluted zinc ions react with a water-soluble polymer such as an organic ultraviolet absorber or a thickener and the like, and this may lead problems such as the deterioration of performance, discoloration, and an increase or decrease in viscosity of the cosmetic products . Therefore, in a case where zinc oxide is used in water-based cosmetic products, unfortunately, a degree of freedom of formulation is limited.

Particularly, in a case where a carbomer such as a carboxyvinyl polymer widely used as a thickener is used in combination with zinc oxide, the eluted zinc ions react with a carboxylate group (COO-) of the carbomer. Consequently, unfortunately, the gel structure of the carbomer is destroyed, and the viscosity of the cosmetic product decreases. Therefore, surface-coated zinc oxide is suggested which is obtained by coating the surface of zinc oxide with an inorganic compound such as silicon oxide so as to inhibit the elution of zinc ions (for example, see Patent Literature Nos. 3 and 4).

Citation List

Patent Literature

[Patent Literature No. 1] Japanese Laid-open Patent Publication No. 3-183620
[Patent Literature No. 2] Japanese Laid-open Patent Publication No. 11-256133
[Patent Literature No. 3] Japanese Laid-open Patent Publication No. 11-302015
[Patent Literature No. 4] Japanese Laid-open Patent Publication No. 2007-16111

Summary of Invention

Technical Problem

However, even in a case where the surface-coated zinc oxide described in Patent Literature Nos. 1 to 4 is used in water-based cosmetic products, the effect of inhibiting the decrease in viscosity of the cosmetic products is insufficient and needs to be further improved.

Particularly, in recent years, the content of water in cosmetic products has tended to obtain a texture closer to that of water. In a case where the content of water is increased, the decrease in viscosity of the cosmetic products cannot be inhibited, and the problem of quality deterioration arises. Furthermore, in a case where the content of a thickener is increased to maintain the viscosity of cosmetic products, the fresh feeling may be lost, a salt may be generated because the zinc ions eluted from the surface-coated zinc oxide react with a carbomer, and hence the texture may deteriorate.

The present invention has been made in consideration of the aforementioned circumstances, and an object thereof is to provide silicon oxide-coated zinc oxide which can suppress a decrease in viscosity resulting from a carbomer in a case where the silicon oxide-coated zinc oxide is used in water-based materials, a composition containing silicon oxide-coated zinc oxide, and a cosmetic product.

Solution to Problem

In order to achieve the above object, the inventors of the present invention repeated intensive examination, and as a result, they made the following invention. That is, the inventors obtained knowledge that for silicon oxide-coated zinc oxide containing zinc oxide particles and a silicon oxide coating on a surface of the zinc oxide particles, by controlling an electric conductivity of a water suspension of the silicon oxide-coated zinc oxide at 120 $\mu$S/cm or less, even in a case where the silicon oxide-coated zinc oxide is used in water-based materials such as water-based cosmetic products, a decrease in viscosity resulting from a carbomer can be suppressed, and the quality stability of the water-based materials can be maintained. Based on the knowledge, the inventors accomplished the present invention.

Silicon oxide-coated zinc oxide of the present invention contains a zinc oxide particle and a silicon oxide coating on a surface of the zinc oxide particle, in which an electric conductivity of a water suspension of the silicon oxide-coated zinc oxide is 120 $\mu$S/cm or less.

A composition containing silicon oxide-coated zinc oxide of the present invention contains the silicon oxide-coated zinc oxide of the present invention.

A cosmetic product of the present invention contains the composition containing silicon oxide-coated zinc oxide of the present invention.

Advantageous Effects of Invention

The electric conductivity of a water suspension of the silicon oxide-coated zinc oxide of the present invention is 120 $\mu$S/cm or less. Therefore, even in a case where the silicon oxide-coated zinc oxide is used in water-based materials such as water-based cosmetic products, a decrease in viscosity resulting from a carbomer can be suppressed, and the quality stability of the water-based materials can be maintained.

The composition containing silicon oxide-coated zinc oxide of the present invention contains the silicon oxide-coated zinc oxide of the present invention. Therefore, even in a case where the composition is used in water-based materials such as water-based cosmetic products, a decrease in viscosity resulting from a carbomer can be suppressed, and the quality stability of the water-based materials can be maintained.

The cosmetic product of the present invention contains the composition containing silicon oxide-coated zinc oxide of the present invention. Therefore, in the cosmetic product, a decrease in viscosity resulting from a carbomer does not occur, and the cosmetic product has excellent quality stability.

Brief Description of Drawings

FIG. 1 is a graph showing a temporal change in viscosity of the composition containing silicon oxide-coated zinc oxide of Examples 1 to 4 and Comparative Example 1.

FIG. 2 is an electron micrograph showing silicon oxide-coated zinc oxide of Example 3.

Description of Embodiments

Preferred embodiments for embodying silicon oxide-coated zinc oxide, a composition containing silicon oxide-coated zinc oxide, and a cosmetic product containing the composition of the present invention will be described.

The following embodiments are specific descriptions for promoting understanding of the gist of the present invention, and unless otherwise specified, the present invention is not limited to the embodiments.

[Silicon oxide-coated zinc oxide]

The silicon oxide-coated zinc oxide of the present embodiment contains a zinc oxide particle and a silicon oxide coating on a surface of the zinc oxide particle, in which an electric conductivity of a water suspension of the silicon oxide-coated zinc oxide is 120 $\mu$S/cm or less.

The electric conductivity of a water suspension of the silicon oxide-coated zinc oxide of the present embodiment means a value measured by the following method.

10 g of silicon oxide-coated zinc oxide and 90 g of pure water are mixed together, and while being stirred, the mixed solution is boiled for 10 minutes on a hot plate. Thereafter, the mixed solution is left to cool to 25°C, and then pure water is added to the mixed solution such that the total amount of the silicon oxide-coated zinc oxide and the pure water becomes 100 g, thereby obtaining a suspension. The electric conductivity of the water suspension is measured using a conductivity meter (trade name: PERSONAL SC METER SC 72, manufactured by Yokogawa Electric Corporation).

The electric conductivity of the water suspension of the silicon oxide-coated zinc oxide of the present embodiment is preferably low, and most preferably 0 $\mu$S/cm. In contrast, because it is difficult to remove all the impurities contained in the silicon oxide-coated zinc oxide, the electric conductivity of the water suspension of the silicon oxide-coated zinc oxide of the present embodiment is preferably 10 $\mu$S/cm or more and 110 uS/cm or less, more preferably 20 $\mu$S/cm or more and 100 $\mu$S/cm or less, and even more preferably 30 $\mu$S/cm or more and 90 $\mu$S/cm or less.

The method for controlling the electric conductivity of the water suspension of the silicon oxide-coated zinc oxide within the aforementioned range, that is, the method for reducing the electric conductivity of the water suspension of the silicon oxide-coated zinc oxide is not particularly limited. Examples thereof include a method of reinforcing cleaning of the silicon oxide-coated zinc oxide in the manufacturing process, a method of appropriately adjusting the manufacturing condition such that the amount of impurities remaining in the silicon oxide-coated zinc oxide is reduced, and the like.

Examples of the method of reinforcing cleaning of the silicon oxide-coated zinc oxide include a method of using a device having a high cleaning power, increasing the number of times of cleaning, and the like.

Examples of solvents used for cleaning include water, alcohols, and the like.

The silicon oxide-coated zinc oxide of the present embodiment is not particularly limited as long as the electric conductivity of the water suspension thereof is 120 $\mu$S/cm or less. For example, it is preferable to use silicon oxide-coated zinc oxide containing zinc oxide particles and a dense silicon oxide coating on a surface of the zinc oxide particles, or silicon oxide-coated zinc oxide in which an alkali metal remaining in the silicon oxide-coated zinc oxide is substituted with at least one element selected from the group consisting of Mg, Ca, and Ba.

(Example 1 of silicon oxide-coated zinc oxide)

As an example of the silicon oxide-coated zinc oxide having a dense silicon oxide coating, there is silicon oxide-coated zinc oxide containing a zinc oxide particle and a silicon oxide coating on a surface of the zinc oxide particle, in which provided that an abundance ratio of silicon in the silicon oxide coating in a $Q^3$ environment is $Q^3$ and that an abundance ratio thereof in a $Q^4$ environment is $Q^4$, $Q^3 + Q^4 \geq 0.6$ and $Q^4/(Q^3 + Q^4) \geq 0.5$. Furthermore, it is preferable that the whole of the zinc oxide particle is evenly coated with the silicon oxide coating such that a decomposition rate of brilliant blue resulting from the photocatalytic activity of the zinc oxide particles becomes 3% or less.

It is preferable that the silicon oxide coating has a high degree of condensation so as to satisfy the condition of "provided that an abundance ratio of silicon in a $Q^3$ environment is $Q^3$ and that an abundance ratio thereof in a $Q^4$ environment is $Q^4$, $Q^3 + Q^4 \geq 0.6$ and $Q^4/(Q^3 + Q^4) \geq 0.5$".

There is a close correlation between "denseness" of the dense silicon oxide coating and "degree of condensation" of silicon oxide. The higher the degree of condensation of silicon oxide, the higher the denseness of the silicon oxide coating.

That is, "dense" for the dense silicon oxide coating mentioned herein means that the degree of condensation of silicon oxide is high such that the conditions of $Q^3 + Q^4 \geq 0.6$ and $Q^4/(Q^3 + Q^4) \geq 0.5$ are satisfied, that is, the values of $Q^3 + Q^4$ and $Q^4/(Q^3 + Q^4)$ are greater than the lower limit.

The degree of condensation of silicon oxide can be easily ascertained by measuring an NMR spectrum of the silicon oxide-coated zinc oxide by a solid-state $^{29}$Si MAS-nuclear magnetic resonance (NMR) spectroscopy and measuring an area ratio of a signal attributed to each of $Q^0$, $Q^1$, $Q^2$, $Q^3$, and $Q^4$ environments from a peak area ratio of the NMR spectrum.

Herein, $Q^n$ (n = 0 to 4) represents cross-linked oxygen atoms among oxygen atoms of a $SiO_4$ tetrahedron unit which is a constitutional unit of silicon oxide, that is, a chemical structure determined according to the number of oxygen atoms bonded to two Si atoms.

The area ratio of the signal attributed to each of the $Q^0$, $Q^1$, $Q^2$, $Q^3$, and $Q^4$ environments is denoted by $Q^0$, $Q^1$, $Q^2$, $Q^3$, and $Q^4$. Here, $Q^0 + Q^1 + Q^2 + Q^3 + Q^4 = 1$.

The decomposition rate of brilliant blue resulting from the photocatalytic activity of the zinc oxide particles is preferably 3% or less for the following reason. In a case where the decomposition rate of brilliant blue is 3% or less, it implies that the photocatalytic activity of the zinc oxide particles is suppressed, and this means that the homogeneity of the silicon oxide coating covering the zinc oxide particle is high. Herein, "the homogeneity of the silicon oxide coating covering the zinc oxide particle is high" means that coating unevenness is not found, the coating is not localized, and a pinhole or the like does not exist. The decomposition rate of brilliant blue is used as a parameter of the photocatalytic activity of the zinc oxide particle. Basically, a photocatalytic reaction of the zinc oxide particle occurs on the surface of the zinc oxide particle. That is, in a case where the decomposition rate of brilliant blue resulting from the photocatalytic activity of the zinc oxide particle is low, this means that the number of sites where the zinc oxide particle is exposed within the surface of the silicon oxide-coated zinc oxide is small.

The decomposition rate of brilliant blue is measured by the following method.

First, an aqueous brilliant blue solution is prepared in which a content rate of brilliant blue is adjusted to become a predetermined value (for example, 5 ppm), and from the aqueous brilliant blue solution, a predetermined amount of solution is collected into a screw pipe. Into the collected aqueous brilliant blue solution, silicon oxide-coated zinc oxide, which is in an amount of 1% by mass in terms of zinc oxide based on the mass of the solution, is added and dispersed using ultrasonic waves, thereby preparing a suspension. Then, the suspension is irradiated with ultraviolet rays having a predetermined wavelength from a predetermined distance (for example, 10 cm) for a predetermined time (for example, 6 hours).

As a lamp for ultraviolet irradiation, for example, a germicidal lamp GL20 (wavelength: 253.7 nm, power of ultraviolet rays: 7.5 W: manufactured by TOSHIBA CORPORATION) can be used.

Then, supernatant liquid is collected from the suspension irradiated with ultraviolet rays, and by atomic absorption spectroscopy, an absorption spectroscopic spectrum of each of the aqueous brilliant blue solution and the supernatant liquid is measured. By using the measured value, a decomposition rate D of brilliant blue is calculated by Equation (1).

$$D = (A0 - A1)/A0 \cdots (1)$$

(A0 represents an absorbance of the aqueous brilliant blue solution (5 ppm) at an absorption maximum wavelength (630 nm) in the absorption spectroscopic spectrum, and A1 represents an absorbance of the supernatant liquid at an absorption maximum wavelength in the absorption spectroscopic spectrum.)

For general zinc oxide (average particle diameter: 35 nm; manufactured by SUMITOMO OSAKA CEMENT Co., Ltd.), the decomposition rate of brilliant blue was measured based on the aforementioned method, and as a result, the decomposition rate was 90%. Therefore, it was confirmed that in a case where the zinc oxide (average particle diameter: 35 nm; manufactured by SUMITOMO OSAKA CEMENT Co., Ltd.) has a photocatalytic activity, the decomposition rate of brilliant blue is high.

The average particle diameter of the silicon oxide-coated zinc oxide is preferably 3 nm or more and 2 $\mu$m or less. In order for a cosmetic product to have intended transparency and ultraviolet shielding properties, the average particle diameter is appropriately adjusted within the aforementioned range. In a case where a highly transparent cosmetic product is required, the average particle diameter of the silicon oxide-coated zinc oxide is preferably 3 nm or more and 50 nm or less. In contrast, in a case where the improvement of the ultraviolet shielding properties of a cosmetic product is required, the average particle diameter of the silicon oxide-coated zinc oxide is preferably 50 nm or more and 2 $\mu$m or less.

In the present embodiment, "average particle diameter" is a numerical value determined by the following method.

That is, the silicon oxide-coated zinc oxide of the present embodiment is observed using a transmission electron microscope (TEM) or the like, and in this case, a predetermined number of silicon oxide-coated zinc oxide particles, for example, two hundred or one hundred particles are selected. Then, the length of a longest straight-line portion (maximum major axis) of the silicon oxide-coated zinc oxide particles is measured, and a weighted average thereof is calculated.

In a case where the silicon oxide-coated zinc oxide particles agglomerate together, the diameter of the agglomerated particle of the agglomerate is not measured. In this case, the diameter of a predetermined number of particles (primary particles) of the silicon oxide-coated zinc oxide constituting the agglomerate is measured and taken as an average particle diameter.

The content of the zinc oxide particles in the silicon oxide-coated zinc oxide is preferably 50% by mass or more and 90% by mass or less. In a case where the content of the zinc oxide particles in the silicon oxide-coated zinc oxide is less than 50% by mass, an intended ultraviolet shielding effect is not obtained. In a case where it is required to obtain an intended ultraviolet shielding effect, a large amount of silicon oxide-coated zinc oxide should be used. Therefore, it is not preferable that the content of the zinc oxide particles is less than 50% by mass. In contrast, in a case where the content of the zinc oxide particles in the silicon oxide-coated zinc oxide is more than 90% by mass, the proportion of the zinc oxide particles in the silicon oxide-coated zinc oxide becomes too high. As a result, the surface of the zinc oxide particles cannot be sufficiently covered with the silicon oxide coating. Therefore, it is not preferable that the content of the zinc oxide particles is more than 90% by mass.

In a case where the silicon oxide-coated zinc oxide in an amount of 0.05% by mass is immersed for 1 hour in an aqueous solution with a hydrogen ion exponent (pH) of 5, an elution rate of zinc eluted into the aqueous solution is preferably 60% by mass or less, more preferably 20% by mass or less, and even more preferably 10% by mass or less.

It is preferable that the elution rate of zinc is 60% by mass or less for the following reason. In a case where the elution rate of zinc is more than 60% by mass, the stability of the silicon oxide-coated zinc oxide itself deteriorates. In a case where the silicon oxide-coated zinc oxide is used in cosmetic products, the eluted zinc ions react with a water-soluble polymer such as an organic ultraviolet shielding agent or a thickener and the like. As a result, the deterioration of the performance of the cosmetic products, discoloration, an increase or decrease in viscosity, and the like occur. Therefore, it is not preferable that the elution rate of zinc is more than 60% by mass.

The elution rate of zinc can be measured by, for example, dispersing the silicon oxide-coated zinc oxide in an amount of 0.05% by mass in a buffer solution with a pH of 5, stirring the solution for 1 hour, then performing solid-liquid separation, and measuring the concentration of zinc in the liquid phase by using an ICP emission spectrometer.

The buffer solution with a pH of 5 is not particularly limited as long as the silicon oxide-coated zinc oxide can be dispersed in the buffer solution. For example, a buffer solution is suitably used which is obtained by mixing 500 ml of a 0.1 M aqueous potassium hydrogen phthalate solution with 226 ml of a 0.1 M aqueous sodium hydroxide solution and then adding water thereto to obtain a total 1,000 ml of a buffer solution.

In order for a cosmetic product to have intended transparency and ultraviolet shielding properties, the average particle diameter of the zinc oxide particles is appropriately adjusted. In a case where a highly transparent cosmetic product is required, the average particle diameter of the zinc oxide particles is preferably 1 nm or more and 50 nm or less. In contrast, in a case where the improvement the ultraviolet shielding properties of a cosmetic product is required, the average particle diameter of the zinc oxide particles is preferably 50 nm or more and 500 nm or less.

The method for manufacturing the silicon oxide-coated zinc oxide is described in detail in International Publication No. WO2014/171322. According to this manufacturing method, the surface of zinc oxide of zinc oxide particles is coated with a silicon oxide coating by using alkoxysilane or using sodium silicate and alkoxysilane, followed by calcination at 200°C to 600°C, thereby obtaining silicon oxide-coated zinc oxide.

In a case where zinc oxide particles having an average particle diameter of 50 nm or more are used, calcination may be performed at 150°C to 600°C.

(Example 2 of silicon oxide-coated zinc oxide)

As another example of the silicon oxide-coated zinc oxide, there is silicon oxide-coated zinc oxide containing a zinc oxide particle, a silicon oxide coating on a surface of the zinc oxide particle, and at least one element selected from the group consisting of Mg, Ca, and Ba. It is preferable to use the aforementioned silicon oxide-coated zinc oxide for the following reason.

In order to evenly coat the entire surface of the zinc oxide particles with a silica coating such that the decomposition rate of brilliant blue resulting from the photocatalytic activity of the zinc oxide particles becomes 3% or less, it is preferable to form a silicon oxide coating by using a material containing an alkali metal such as sodium silicate. However, in a case where the alkali metal remains in the silicon oxide-coated zinc oxide, when the silicon oxide-coated zinc oxide is dispersed in a water phase, alkali ions are eluted. As a result, the pH or viscosity greatly changes, and hence the quality stability of a cosmetic product deteriorates.

In a case where the alkali metal contained in the silicon oxide coating of the silicon oxide-coated zinc oxide is substituted with at least one element selected from the group consisting of Mg, Ca, and Ba, the alkali metal contained in the silicon oxide coating of the silicon oxide-coated zinc oxide is removed from the silicon oxide coating of the silicon oxide-coated zinc oxide.

After substitution, at least one element selected from the group consisting of Mg, Ca, and Ba that substitutes the alkali metal contained in the silicon oxide coating exists in the silicon oxide coating of the silicon oxide-coated zinc oxide. Mg, Ca, and Ba used for substitution exist as magnesium silicate, calcium silicate, barium silicate, and the like that exhibit low solubility in water.

As a result of substitution, a total mass percentage of Mg, Ca, and Ba contained in the silicon oxide coating of the silicon oxide-coated zinc oxide becomes larger than a total mass percentage of the alkali metal contained in the silicon oxide coating. Therefore, even though the silicon oxide-coated zinc oxide is mixed with a water phase, the elution of the alkali metal is inhibited. Accordingly, the change in the pH or viscosity can be inhibited, and the quality stability of a cosmetic product can be maintained.

The average particle diameter of the silicon oxide-coated zinc oxide is selected as necessary, but is preferably 2 nm or more and 2 $\mu$m or less, more preferably 5 nm or more and 500 nm or less, and even more preferably 10 nm or more and 400 nm or less.

The smaller the average particle diameter of the silicon oxide-coated zinc oxide is, the more the silicon oxide-coated zinc oxide is suitable for improving transparency at the time of use in a case where the silicon oxide-coated zinc oxide is mixed with a cosmetic product. In contrast, the greater the average particle diameter of the silicon oxide-coated zinc oxide is, the higher the ultraviolet scattering power, and the ultraviolet rays having a long wavelength can also be shielded. Therefore, the average particle diameter of the silicon oxide-coated zinc oxide is appropriately selected according to the transparency and ultraviolet shielding properties of a cosmetic product of interest.

The content rate of the zinc oxide particle in the silicon oxide-coated zinc oxide is selected as necessary, but is preferably 50% by mass or more and 99% by mass or less, more preferably 70% by mass or more and 95% by mass or less, and even more preferably 70% by mass or more and 90% by mass or less.

In a case where the content rate of the zinc oxide particle in the silicon oxide-coated zinc oxide is less than 50% by mass, an intended ultraviolet shielding effect is unlikely to be obtained. For a cosmetic product containing the aforementioned silicon oxide-coated zinc oxide in a cosmetic base material, in a case where it is required to obtain an intended ultraviolet shielding effect, a large amount of silicon oxide-coated zinc oxide should be used. Therefore, it is not preferable that the content rate of the zinc oxide particle is less than 50% by mass.

In contrast, in a case where the content rate of the zinc oxide particle in the silicon oxide-coated zinc oxide is more than 99% by mass, the proportion of the zinc oxide particle in the silicon oxide-coated zinc oxide is likely to be too high. As a result, the surface of the zinc oxide particle cannot be sufficiently covered with the silicon oxide coating, and the photocatalytic activity of zinc oxide or the elution of zinc ions is unlikely to be sufficiently inhibited. Therefore, it is not preferable that the content rate of the zinc oxide particle is more than 99% by mass.

The content rate of silicon oxide in the silicon oxide-coated zinc oxide is appropriately adjusted according to the average particle diameter of the zinc oxide particles. For example, for the zinc oxide particles having an average particle diameter of 50 nm or less, the content rate of silicon oxide is preferably 3% by mass or more and 45% by mass or less. For the zinc oxide particles having an average particle diameter of more than 50 nm, the content rate of silicon oxide is preferably 1% by mass or more and 35% by mass or less.

The silicon oxide-coated zinc oxide contains at least one element selected from the group consisting of Mg, Ca, and Ba.

In the silicon oxide-coated zinc oxide, a total mass percentage of Mg, Ca, and Ba contained in the silicon oxide coating is preferably larger than the total mass percentage of the alkali metal contained in the silicon oxide coating. Furthermore, a ratio of the total mass percentage of the alkali metal contained in the silicon oxide coating to the total mass percentage of Mg, Ca, and Ba contained in the silicon oxide coating (total mass percentage of alkali metal/total mass percentage of Mg, Ca, and Ba) is preferably 0.001 or more and 0.6 or less, more preferably 0.01 or more and 0.5 or less, and even more preferably 0.1 or more and 0.4 or less.

In the present embodiment, the alkali metal refers to generally known one, and specifically means at least one metal selected from the group consisting of lithium, sodium, potassium, rubidium, cesium, and francium.

The hydrogen ion exponent (pH) of the silicon oxide-coated zinc oxide at the initial stage is changed not by the elution of zinc ions but mainly by the elution of the alkali metal ions contained in the silicon oxide coating. For this reason, the total mass percentage of Mg, Ca, and Ba contained in the silicon oxide coating is made larger than the total mass percentage of the alkali metal contained in the silicon oxide coating.

The total mass percentage of the alkali metal contained in the silicon oxide coating in the silicon oxide-coated zinc oxide is preferably 0.2% by mass or less, and more preferably 0.15% by mass or less.

The lower limit of the total mass percentage of the alkali metal contained in the silicon oxide coating can be arbitrarily selected. The total mass percentage of the alkali metal may be 0% by mass, or may be 0.0001% by mass or more, 0.001% by mass or more, and the like, for example.

The total mass percentage of Mg, Ca, and Ba contained in the silicon oxide coating in the silicon oxide-coated zinc oxide is preferably 0.01% by mass or more and 1% by mass or less.

The mass percentage (% by mass) of the alkali metal, Mg, Ca, and Ba contained in the silicon oxide-coated zinc oxide (silicon oxide coating) can be measured by atomic absorption spectroscopy.

In the silicon oxide-coated zinc oxide, the decomposition rate of brilliant blue resulting from the photocatalytic activity of the zinc oxide particles is preferably 3% or less, more preferably 2% or less, and even more preferably 1% or less.

Furthermore, it is preferable that the silicon oxide coating of the silicon oxide-coated zinc oxide satisfies the condition of "provided that an abundance ratio of silicon in the silicon oxide coating in a $Q^3$ environment is $Q^3$ and that an abundance ratio thereof in a $Q^4$ environment is $Q^4$, $Q^3 + Q^4 \geq 0.6$ and $Q^4/(Q^3 + Q^4) \geq 0.5$."

[Method for manufacturing silicon oxide-coated zinc oxide]

The method for manufacturing silicon oxide-coated zinc oxide of the present embodiment will be described.

The method for manufacturing silicon oxide-coated zinc oxide of the present embodiment is a manufacturing method including a step of mixing together composite particles obtained by coating a surface of a zinc oxide particle with silicon oxide containing an alkali metal, at least one element selected from the group consisting of Mg, Ca, and Ba, and a solution containing water, and substituting the alkali metal contained in the silicon oxide with at least one element selected from the group consisting of Mg, Ca, and Ba (hereinafter, referred to as "substitution step"); and a calcination step.

The manufacturing method may include a step of forming a silicon oxide coating having a higher degree of condensation by adding zinc oxide coated with silicon oxide containing an alkali metal which is not yet been subjected to the substitution step or silicon oxide-coated zinc oxide having undergone the substitution step that contains at least one element selected from the group consisting of Mg, Ca, and Ba; at least one compound selected from the group consisting of an alkoxysilane and an alkoxysilane oligomer having 10 or less monomer; a catalyst; and water, and stirring the mixture for 30 minutes or longer and 24 hours or shorter to cause a reaction.

Next, the method for manufacturing silicon oxide-coated zinc oxide will be specifically described.

As the zinc oxide coated with silicon oxide containing an alkali metal, a compound may be used which is obtained by reacting silicate containing an alkali metal such as sodium silicate with zinc oxide particles such that the surface of the zinc oxide particle is coated with silicon oxide. Alternatively, commercially available zinc oxide coated with silicon oxide may be used.

As the method for coating the surface of the zinc oxide particle with silicon oxide, for example, it is possible to use the methods described in Japanese Laid-open Patent Publication No.03-183620, Japanese Laid-open Patent Publication No. 11-256133, Japanese Laid-open Patent Publication No. 11-302015, Japanese Laid-open Patent Publication No. 2007-016111, and the like.

The method for coating the surface of the zinc oxide particle with silicon oxide is selected as necessary, and examples thereof include the following method.

First, zinc oxide particles and water are mixed together, and then the zinc oxide particles are dispersed in water by using ultrasonic waves, thereby preparing a water-based zinc oxide suspension.

Thereafter, the water-based zinc oxide suspension is heated, an aqueous sodium silicate solution is added to the water-based zinc oxide suspension with stirring, and the suspension is matured for 10 minutes to 60 minutes.

Then, while the water-based zinc oxide suspension is being stirred, an acid such as dilute sulfuric acid is added thereto such that the pH is adjusted and becomes 5 to 9, and the suspension is matured for 30 minutes to 5 hours.

Subsequently, the reaction solution is subjected to solid-liquid separation, the obtained reaction product is cleaned with a solvent such as water and then dried at a temperature of about 100°C to 200°C, thereby obtaining zinc oxide particle coated with silicon oxide containing an alkali metal.

[Substitution step]

The substitution step needs to be performed after the step of coating the surface of the zinc oxide particles with silicon oxide containing an alkali metal. This is because in a case where silicate containing an alkali metal is mixed with at least one element selected from the group consisting of Mg, Ca, and Ba simply in a solution containing water, a precipitate of at least one compound among magnesium silicate, calcium silicate, and barium silicate is generated as impurity. Therefore, by causing a neutralization reaction of the silicate or the like, the substitution step is preferably incorporated into a stage anywhere between a point in time when the step of coating the surface of the zinc oxide particle with silicon oxide ends and a point in time when the drying step ends. According to this method, the reaction process can be reduced. Therefore, it is possible to obtain the silicon oxide-coated zinc oxide of the present embodiment at low costs.

In the substitution step, first, the zinc oxide coated with silicon oxide containing an alkali metal and at least one element selected from the group consisting of Mg, Ca, and Ba are added to a solution containing water and mixed together.

The solution containing water is not particularly limited, and is selected as necessary. As the solution containing water, for example, water or a solution obtained by mixing water with a solvent compatible with water is used.

As the solvent compatible with water, for example, a protic polar solvent such as methanol, ethanol, or 2-propanol and an aprotic polar solvent such as acetone or tetrahydrofuran are preferable. Among these, a protic polar solvent such as methanol, ethanol, or 2-propanol is more preferable.

The reaction temperature at the aforementioned mixing treatment is not particularly limited, and is adjusted as necessary. The reaction temperature may be equal to or higher than a solidifying point of the solvent in the mixed solution containing the zinc oxide coated with silicon oxide, at least one element selected from the group consisting of Mg, Ca, and Ba, and the solution containing water.

Although the reaction proceeds in a state where the mixed solution is allowed to stand, in order to increase the reaction efficiency, it is preferable to perform the reaction with stirring the mixed solution.

The reaction time is not particularly limited and is selected as necessary. The reaction time is preferably 1 hour or longer.

By the mixing treatment, the alkali metal in the zinc oxide coated with silicon oxide is substituted with at least one element selected from the group consisting of Mg, Ca, and Ba, and eluted into the mixed solution from the zinc oxide coated with silicon oxide. In contrast, at least one ion selected from the group consisting of Mg, Ca, and Ba substituting the alkali metal is incorporated into the silicon oxide-coated zinc oxide due to the substitution with the alkali metal. As a result, silicon oxide-coated zinc oxide containing at least one element selected from the group consisting of Mg, Ca, and Ba is obtained.

The content of at least one element selected from the group consisting of Mg, Ca, and Ba that is contained in the mixed solution is not particularly limited, and is selected as necessary. In order to exchange the alkali ion such as Na or K in the zinc oxide coated with silicon oxide with at least one ion selected from the group consisting of Mg, Ca, and Ba, the content of at least one element selected from the group consisting of Mg, Ca, and Ba that is contained in the mixed solution is preferably equal to or greater than the sum of molar equivalents of the alkali metals in the zinc oxide coated with silicon oxide.

The raw material providing at least one element selected from the group consisting of Mg, Ca, and Ba is not particularly limited as long as the raw material is an inorganic salt contains these elements. Examples of the raw material providing Mg include magnesium chloride, magnesium sulfate, magnesium nitrate, and the like. Examples of the raw material providing Ca include calcium chloride, calcium nitrate, and the like. As the raw material providing Ba, for example, barium chloride, barium nitrate, and the like are suitably used.

These raw materials may be used as they are in the form of solid or may be used after being made into an aqueous solution.

Thereafter, the mixed solution containing the silicon oxide-coated zinc oxide generated by the substitution step is subjected to solid-liquid separation by filtration under normal pressure, filtration under reduced pressure, filtration under increased pressure, centrifugation, and the like. By cleaning the obtained solid with a solvent such as water, silicon oxide-coated zinc oxide is obtained.

In order to further reduce the content of the alkali metal in the obtained silicon oxide-coated zinc oxide, after the solid-liquid separation, it is preferable to a step of mixing the obtained silicon oxide-coated zinc oxide again with at least one element selected from the group consisting of Mg, Ca, and Ba in the solution containing water and substituting the alkali metal in the silicon oxide-coated zinc oxide with at least one element selected from the group consisting of Mg, Ca, and Ba. It is more preferable to repeat the substitution step plural times.

The silicon oxide-coated zinc oxide obtained in this way contains water. Therefore, in order to remove water, it is preferable to dry the silicon oxide-coated zinc oxide.

The drying temperature is not particularly limited. However, generally, it is preferable to dry the silicon oxide-coated zinc oxide at a temperature of 100°C or more. In a case where the silicon oxide-coated zinc oxide is dried at a temperature of 80°C or lower, the drying is preferably performed under reduced pressure.

Then, the dried substance is subjected to a thermal treatment (calcination) at a temperature of 200°C or more and less than 600°C, and in this way, the silicon oxide-coated zinc oxide of the present embodiment can be prepared.

[Composition containing silicon oxide-coated zinc oxide]

The composition containing silicon oxide-coated zinc oxide of the present embodiment contains the silicon oxide-coated zinc oxide of the present embodiment.

In the composition containing silicon oxide-coated zinc oxide of the present embodiment, the average particle diameter of silicon oxide-coated zinc oxide can be arbitrarily selected, but is preferably 2 nm or more and 500 nm or less, more preferably 5 nm or more and 400 nm or less, and even more preferably 10 nm or more and 400 nm or less.

The average particle diameter of the silicon oxide-coated zinc oxide is limited to the aforementioned range for the following reason. In a case where the average particle diameter is less than 2 nm, because the particle diameter is too small, the surface energy of the silicon oxide-coated zinc oxide is high. Therefore, the particles easily agglomerate together, and it is difficult to maintain intended shape and size. In a case where the average particle diameter is larger than 500 nm, the transparency of the silicon oxide-coated zinc oxide easily deteriorates. Accordingly, in a case where the composition containing silicon oxide-coated zinc oxide is used in a cosmetic product and the like, the transparency in the visible ray region may deteriorate, or the texture may deteriorate due to the occurrence of squeakiness or the like.

The average dispersed-particle diameter of the silicon oxide-coated zinc oxide in the composition containing silicon oxide-coated zinc oxide of the present embodiment is preferably 10 nm or more and 1 $\mu$m or less, more preferably 20 nm or more and 800 nm or less, and even more preferably 25 nm or more and 500 nm or less. In a case where the average dispersed-particle diameter of the silicon oxide-coated zinc oxide is less than 10 nm, the crystallinity of the silicon oxide-coated zinc oxide is reduced, and hence sufficient ultraviolet shielding properties may not be exhibited. In contrast, in a case where the average dispersed-particle diameter of the silicon oxide-coated zinc oxide is more than 1 $\mu$m, glariness, squeakiness, and the like occur. Accordingly, in a case where the silicon oxide-coated zinc oxide is formulated with a cosmetic product, the texture of the cosmetic product at the time of use may become poor, the dispersion stability may deteriorate, and a stabilized composition may not be obtained. In the present invention, the dispersed-particle diameter means a particle diameter in a state where a plurality of silicon oxide-coated zinc oxide particles which agglomerating together are dispersed.

The content rate of the silicon oxide-coated zinc oxide in the composition containing silicon oxide-coated zinc oxide of the present embodiment may be appropriately adjusted for obtaining an intended ultraviolet shielding performance, and is not particularly limited. The content rate of the silicon oxide-coated zinc oxide is preferably 1% by mass or more and 80% by mass or less, more preferably 5% by mass or more and 70% by mass or less, and even more preferably 10% by mass or more and 60% by mass or less. In order to effectively exploit the characteristics of the silicon oxide-coated zinc oxide of the present invention, the content rate of the silicon oxide-coated zinc oxide may be 1% by mass or more and 20% by mass or less. Alternatively, the content rate may be 2% by mass or more and 10% by mass or less or 3% by mass or more and 7% by mass or less.

As described above, the content rate of the silicon oxide-coated zinc oxide is preferably 1% by mass or more and 80% or less. This is because in a case where the content rate of the silicon oxide-coated zinc oxide is less than 1% by mass, the composition containing silicon oxide-coated zinc oxide cannot exhibit a sufficient ultraviolet shielding function. As a result, when the composition containing silicon oxide-coated zinc oxide is mixed with a cosmetic product or the like, in order to make the cosmetic product exhibit an intended ultraviolet shielding function, a large amount of composition containing silicon oxide-coated zinc oxide needs to be added, and this may lead to an increase in manufacturing costs. Therefore, it is not preferable that the content rate of the silicon oxide-coated zinc oxide is less than 1% by mass. In contrast, in a case where the content rate of the silicon oxide-coated zinc oxide is more than 80% by mass, the viscosity of the composition containing silicon oxide-coated zinc oxide increases. As a result, the dispersion stability of the silicon oxide-coated zinc oxide may deteriorate, and hence the silicon oxide-coated zinc oxide may be easily precipitated. Therefore, it is not preferable that the content rate of the silicon oxide-coated zinc oxide is more than 80% by mass.

The solvent used in the composition containing silicon oxide-coated zinc oxide of the present embodiment is not particularly limited as long as it is a solvent in which the silicon oxide-coated zinc oxide can be dispersed.

For example, water; alcohols such as methanol, ethanol, 1-propanol, 2-propanol, 1-butanol, 2-butanol, octanol, and glycerin; esters such as ethyl acetate, butyl acetate, ethyl lactate, propylene glycol monomethyl ether acetate, propylene glycol monoethyl ethyl ether acetate, and γ-butyrolactone; and ethers such as diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, and diethylene glycol monoethyl ether are suitably used. Among these, one solvent may be used singly, or two or more solvents may be used by being mixed together.

As other solvents used in the composition containing silicon oxide-coated zinc oxide of the present embodiment, for example, ketones such as acetone, methyl ethyl ketone, methyl isobutyl ketone, acetyl acetone, and cyclohexanone; aromatic hydrocarbon such as benzene, toluene, xylene, and ethylbenzene; cyclic hydrocarbon such as cyclohexane; amides such as dimethyl formamide, N,N-dimethylacetoacetamide, and N-methylpyrrolidone; and linear polysiloxanes such as diemthyl polysiloxane, methyl phenyl polysiloxane, and diphenyl polysiloxane are also suitably used.

Furthermore, cyclic polysiloxanes such as octamethylcyclotetrasiloxane, decamethylcyclopentasiloxane, and dodecamethylcyclohexasiloxane; and modified polysiloxanes such as amino-modified polysiloxane, polyether-modified polysiloxane, alkyl-modified polysiloxane, and fluorine-modified polysiloxane are also suitably used. Among these, one solvent may be used singly, or two or more solvents may be used by being mixed together.

The composition containing silicon oxide-coated zinc oxide of the present embodiment may contain generally used additives such as a dispersant, a stabilizer, a water-soluble binder, and a thickener, within a range that does not impair the characteristics of the composition.

As the dispersant, an anionic surfactant, a cationic surfactant, an amphoteric surfactant, a nonionic surfactant, a silane coupling agent such as organoalkoxysilane or organochlorosilane, and modified silicone such as polyether-modified silicone or amino-modified silicone are suitably used. The type or amount of these dispersants may be appropriately selected according to the particle diameter of composite particles or to the type of the dispersion medium of interest. Among the above, one dispersant may be used singly, or two or more dispersants may be used by being mixed together.

As the water-soluble binder, for example, it is possible to use polyvinyl alcohol (PVA), polyvinylpyrrolidone, hydroxycellulose, polyacrylic acid, and the like.

In a case where the composition containing silicon oxide-coated zinc oxide of the present embodiment is used in a cosmetic product, the thickener is not particularly limited as long as it is a thickener used in cosmetic products. For example, a natural water-soluble polymer such as gelatin, casein, collagen, hyaluronic acid, albumin, or starch, a semi-isynthetic polymer such as methyl cellulose, ethyl cellulose, methyl hydroxypropyl cellulose, carboxymethyl cellulose, hydroxymethyl cellulose, hydroxypropyl cellulose, sodium carboxymethyl cellulose, or alginic acid propylene glycol ester, a synthetic polymer such as polyvinyl alcohol, polyvinylpyrrolione, a carbomer (carboxyvinyl polymer), polyacrylate, or polyethylene oxide, inorganic mineral such as bentonite, laponite, hectorite, and the like are suitably used. Among these, one thickener may be used singly, or two or more thickeners may be used in combination.

Among these thickeners, a synthetic polymer is preferable, and a carboxyvinyl polymer (carbomer) is more preferable. The carboxyvinyl polymer also includes those obtained by modifying a portion of a carboxyvinyl polymer, such as an alkyl-modified carboxyvinyl polymer.

In a case where a carboxyvinyl polymer is used as a thickener, the content rate of the carbomer in the composition containing silicon oxide-coated zinc oxide of the present embodiment is preferably 0.0001% by mass or more and 10% by mass or less, and more preferably 0.01% by mass or more and 1% by mass or less.

In a case where the content rate of the carboxyvinyl polymer in the composition containing silicon oxide-coated zinc oxide of the present embodiment is less than 0.0001% by mass, a thickening effect may not be obtained. In contrast, from the viewpoint of use, it is not preferable that the content rate of the carboxyvinyl polymer is more than 10% by mass, because then the viscosity of the composition containing silicon oxide-coated zinc oxide becomes too high.

In a case where the carboxyvinyl polymer is used to effectively use the silicon oxide-coated zinc oxide of the present invention, the content rate of the carboxyvinyl polymer in the composition containing silicon oxide-coated zinc oxide of the present invention may be 0.5% by mass or more and 2.5% by mass or less, 1.0% by mass or more and 2.0% by mass or less, or 1.3% by mass or more and 1.7% by mass or less.

In a case where a carboxyvinyl polymer is used as a thickener, the hydrogen ion exponent (pH) of the composition containing silicon oxide-coated zinc oxide is preferably 5 or more and 9 or less, more preferably 6 or more and 9 or less, and even more preferably 7 or more and 9 or less. In a case where the pH of the composition containing silicon oxide-coated zinc oxide of the present embodiment is within the above range, the temporal change of viscosity and the like can be inhibited.

The aforementioned carboxyvinyl polymer is widely used as a thickener of water-based cosmetic products. However, because the thickening (gelatification) is performed by using the interaction between carboxy groups or carboxylate groups, in a case where there is a zinc ion, the network structure of the carboxyvinyl polymer is destroyed, and hence a constant viscosity cannot be maintained. Accordingly, in a case where zinc oxide is mixed in an amount of several % by mass with an aqueous carboxyvinyl polymer solution having undergone viscosity adjustment, a decrease in viscosity proceeds within several hours in some cases. Furthermore, even in a case where zinc oxide is used which is coated with an inorganic oxide or a resin so as to suppress the surface activity thereof, in many cases, a decrease in viscosity or phase separation proceeds within several hours to several days. Therefore, in a case where the carboxyvinyl polymer and zinc oxide are used in combination, the inhibition or reduction of the decrease in viscosity of the mixture containing the carbomer and zinc oxide becomes an issue.

In a case where the decrease in viscosity of the aqueous carboxyvinyl polymer solution is inhibited using zinc oxide of the related art that is coated with an inorganic oxide or a resin so as to suppress the surface activity thereof, a decrease in viscosity that occurs after the elapse of a certain period of time becomes a bigger problem than the decrease in viscosity at the initial stage.

The decrease in viscosity at the initial stage can be addressed by preliminarily adjusting the viscosity of the aqueous carboxyvinyl polymer solution to a high level. However, in a case where a medium and long term change of the viscosity occurs after the elapse of a certain period of time, the properties of the cosmetic product change at the stage of distribution, and hence the temporal stability deteriorates. Particularly, because the zinc oxide having undergone a surface treatment by using an inorganic oxide or a resin exhibits an elution inhibition effect to some extent, a medium and long term elution of zinc ions may slowly proceed.

In the related art, there is no report relating to the change in viscosity of a composition containing a carboxyvinyl polymer, and if aught there be, it was confirmed that the temporal change in viscosity can be inhibited for only up to about 7 days at room temperature.

Herein, in a case where the silicon oxide-coated zinc oxide of the present embodiment is used in which, provided that an abundance ratio of silicon in the silicon oxide coating in a $Q^3$ environment is $Q^3$ and that an abundance ratio thereof in a $Q^4$ environment is $Q^4$, $Q^3 + Q^4 \geq 0.6$ and $Q^4/(Q^3 + Q^4) \geq 0.5$, and the decomposition rate of brilliant blue resulting from the photocatalytic activity of the zinc oxide particles is 3% or less, a composition containing silicon oxide-coated zinc oxide having excellent quality stability is obtained. That is, in the composition containing silicon oxide-coated zinc oxide of the present embodiment, the silicon oxide-coated zinc oxide is used which has a stronger zinc elution inhibition effect compared to the zinc oxide of the related art coated with an inorganic oxide or a resin. Therefore, even though a carboxyvinyl polymer is used as a thickener, a composition is obtained in which the viscosity decreases a little over time and which has excellent quality stability.

In a case where the composition containing silicon oxide-coated zinc oxide of the present embodiment contains a carboxyvinyl polymer as a thickener, the viscosity is preferably 5 Pa·s or more.

In view of handling, it is not preferable that the viscosity is less than 5 Pa·s, because then a cosmetic product containing the composition containing silicon oxide-coated zinc oxide does not spread well on the skin. The upper limit of the viscosity is not particularly limited and may be appropriately adjusted according to an intended texture. For example, the upper limit of the viscosity is preferably 100 Pa·s or less, more preferably 50 Pa·s or less, and even more preferably 15 Pa·s or less. In order to obtain a fresh texture unique to water-based cosmetic products, the upper limit of the viscosity is preferably 15 Pa·s or less.

In order to make the composition containing silicon oxide-coated zinc oxide have a viscosity of 5 Pa·s or more, an electric conductivity of a water suspension of the silicon oxide-coated zinc oxide may be set to be 120 $\mu$S/cm or less. In contrast, the upper limit of the viscosity of the silicon oxide-coated zinc oxide may be adjusted based on the content of the carboxyvinyl polymer. The larger the content of the carboxyvinyl polymer, the higher the viscosity. Accordingly, the carboxyvinyl polymer may be added by appropriately adjusting the content of thereof so as to obtain an intended texture.

The viscosity of the composition containing silicon oxide-coated zinc oxide of the present embodiment is a value measured using a BII-type rotary viscometer (manufactured by TOKI SANGYO CO., LTD.) under the condition of 20°C and 30 rpm.

In a case where the content rate of the silicon oxide-coated zinc oxide in the composition containing silicon oxide-coated zinc oxide of the present embodiment is 15% by mass, and a coating film having a thickness of 32 $\mu$m is formed using the composition, a transmittance of the coating film with respect to light having a wavelength of 450 nm is preferably 50% or more, more preferably 60% or more, and even more preferably 70% or more.

The transmittance can be determined by forming a coating film having a thickness of 32 $\mu$m by coating a quartz substrate with the composition containing silicon oxide-coated zinc oxide that contains silicon oxide-coated zinc oxide in an amount of 15% by mass by using a bar coater, and measuring a spectral transmittance of the coating film by using an SPF ANALYZER UV-1000S (manufactured by Labsphere). At this time, a component of the composition other than the silicon oxide-coated zinc oxide may be, for example, a solvent such as water.

The method for manufacturing the composition containing silicon oxide-coated zinc oxide of the present embodiment is not particularly limited, as long as the silicon oxide-coated zinc oxide can be dispersed in the aforementioned solvent.

As a dispersion method for the aforementioned dispersion, known dispersion methods can be used. For example, dispersion methods using a stirrer, a beads mill using zirconia beads, a ball mill, a homogenizer, an ultrasonic disperser, a kneader, a triple roll mill, a rotation and revolution mixer, and the like are suitably used.

The time required for the dispersion treatment may need to be long enough to evenly disperse the silicon oxide-coated zinc oxide in the aforementioned solvent.

Next, as specific examples of the composition containing silicon oxide-coated zinc oxide of the present embodiment, (a) silicone resin-based composition containing silicon oxide-coated zinc oxide obtained by dispersing silicon oxide-coated zinc oxide in a silicone resin which is a water-insoluble dispersion medium and (b) water-based composition containing silicon oxide-coated zinc oxide obtained by dispersing silicon oxide-coated zinc oxide in water will be described respectively.

(Silicone resin-based composition containing silicon oxide-coated zinc oxide)

The silicone resin-based composition containing silicon oxide-coated zinc oxide is a silicone resin-based composition obtained by dispersing the aforementioned silicon oxide-coated zinc oxide in a silicone resin. The content rate of the silicon oxide-coated zinc oxide in the composition is preferably 1% by mass or more and 80% by mass or less, more preferably 20% by mass or more and 70% by mass or less, and even more preferably 30% by mass or more and 60% by mass or less.

The surface of the silicon oxide-coated zinc oxide may be treated with a silicone resin.

In a case where the surface of the silicon oxide-coated zinc oxide is treated with a silicone resin, the affinity with an oil phase and particularly with silicone oil is improved. Consequently, the silicon oxide-coated zinc oxide is more easily mixed with a water-in-oil type (W/O type) or oil-in-water type (O/W type) cosmetic product.

That is, in a case where the silicon oxide-coated zinc oxide whose surface is treated with a silicone resin is mixed with an oil phase to obtain a water-in-oil type or oil-in-water type cosmetic product, it is possible to inhibit the elution of zinc ions in the water-in-oil type (W/O type) or oil-in-water type (O/W type) cosmetic product.

The silicone resin used for the surface treatment is not particularly limited as long as it can be used as a cosmetic product. Examples of the silicone resin include methyl hydrogen polysiloxane, dimethyl polysiloxane, methicone, hydrogen dimethicone, triethoxysilylethyl polydimethylsiloxyethyl dimethicone, triethoxysilylethyl polydimethylsiloxyethyl hexyl dimethicone, an (acrylate/tridecyl acrylate/triethoxysilylpropyl methacrylate/dimethicone methacrylat) copolymer, triethoxycaprylylsilane, and the like. Among these, one silicon resin may be used singly, or two or more silicon resins may be used by being mixed together. Alternatively, a copolymer of these may be used.

The silicone resin is not particularly as long as it is a cyclic silicone resin or a linear silicone resin having a structural skeleton represented by Formula (2).

$$(- (Si (CH_3)_2 O-)_X \cdots \qquad (2)$$

(In Formula (2), X is within a range of 1 to 2,000.)

It is preferable that the value of X in the silicone resin is within the aforementioned range, because then the silicone resin can be easily mixed with the aforementioned silicon oxide-coated zinc oxide.

Examples of the silicone resin include dimethyl polysiloxane, methyl phenyl polysiloxane, hexamethylcyclotrisiloxane, octamethylcyclotetrasiloxane, decamethyl pentasiloxane, methyl trimethicone, and the like.

The silicone resin-based composition containing silicon oxide-coated zinc oxide may contain a dispersant.

Examples of the dispersant include polyether-modified silicone, polyglycerin-modified silicone, amino-modified silicone, phenyl-modified silicone, alkyl-modified silicone, carbinol-modified silicone, dimethyl silicone, and the like.

The amount of the dispersant added can be arbitrarily selected, but is preferably within a range of 1% by mass or more and 50% by mass or less with respect to the mass of the silicon oxide-coated zinc oxide in the silicone resin-based composition containing silicon oxide-coated zinc oxide. For example, if necessary, the amount of the dispersant added may be within a range of 3% by mass or more and 15% by mass or less, a range of 10% by mass to 30% by mass, or the like.

By adjusting the amount of the dispersant added within the aforementioned range, even in a case where the silicone resin-based composition containing silicon oxide-coated zinc oxide is used singly or directly mixed with a cosmetic product, when the composition is applied and spread onto the skin, transparency can be sufficiently secured.

Furthermore, natural oil, a moisturizer, a thickener, an aromatic, a preservative, and the like may be mixed with the silicone resin-based composition containing silicon oxide-coated zinc oxide, within a range that does not impair the characteristics of the composition.

(Water-based composition containing silicon oxide-coated zinc oxide)

The water-based composition containing silicon oxide-coated zinc oxide is a water-based composition obtained by dispersing the aforementioned silicon oxide-coated zinc oxide in a water-based dispersion medium or a water-based dispersion medium containing alcohols. The content rate of the silicon oxide-coated zinc oxide in this composition is preferably 1% by mass or more and 80% by mass or less, more preferably 20% by mass or more and 70% by mass or less, and even more preferably 30% by mass or more and 60% by mass or less.

Furthermore, in order to effectively exploit the characteristics of the silicon oxide-coated zinc oxide of the present invention, the content rate of the silicon oxide-coated zinc oxide may be 1% by mass or more and 20% by mass or less. Alternatively, the content rate of the silicon oxide-coated zinc oxide may be 2% by mass or more and 10% by mass or less or 3% by mass or more and 7% by mass or less.

The content rate of the water-based dispersion medium or the water-based dispersion medium containing alcohols is preferably 20% by mass to 99% by mass, more preferably 30% by mass to 80% by mass, and even more preferably 40% by mass to 70% by mass.

In addition, in order to effectively exploit the characteristics of the silicon oxide-coated zinc oxide of the present invention, the content rate of the water-based dispersion medium containing alcohols or of water may be 80% by mass to 99% by mass or 90% by mass to 97% by mass.

The water-based dispersion medium means water or water of which the pH is adjusted using an acid or alkali.

The water-based dispersion medium containing alcohols is a dispersion medium containing alcohols and water. Water may be used after the pH thereof is adjusted using an acid or alkali. Examples of the alcohols include a monol or polyol having 1 to 6 carbon atoms, such as ethanol, 1-propanol, 2-propanol, 1-butanol, 2-butanol, octanol, glycerin, 1,3-butylene glycol, propylene glycol, and sorbitol. Among these, a monol is preferable, and ethanol is particularly preferable.

In a case where the water-based composition containing silicon oxide-coated zinc oxide is constituted with the water-based dispersion medium containing the aforementioned silicon oxide-coated zinc oxide and alcohols, the content rate of the alcohols is preferably 5% by mass or more and 20% by mass or less, and more preferably 10% by mass or more and 20% by mass or less.

Particularly, it is preferable that the content rate of the alcohols is 5% by mass or more and 20% by mass or less, because then the dispersibility and the temporal stability of the water-based composition of the silicon oxide-coated zinc oxide can be improved.

The water-based composition containing silicon oxide-coated zinc oxide can further contain a water-soluble polymer preferably in an amount of 0.001% by mass or more and 10% by mass or less, more preferably in an amount of 0.005% by mass or more and 5% by mass or less, and even more preferably in an amount of 0.01% by mass or more and 3% by mass or less. In this case, the content rate of each component needs to be adjusted, lest the total content rate of the silicon oxide-coated zinc oxide, the water-based dispersion medium, the water-based dispersion medium containing alcohols, and the water-soluble polymer becomes more than 100% by mass.

In a case where the water-based composition containing silicon oxide-coated zinc oxide is used in a cosmetic product, the water-soluble polymer contained in the water-based composition is not particularly limited as long as it can be used for cosmetic products. Examples of the water-soluble polymer include gum Arabic, sodium alginate, casein, carrageenan, galactan, a carboxyvinyl polymer, a carboxymethyl cellulose, sodium carboxymethyl cellulose, carboxymethyl starch, agar, quince seed, xanthan gum, guar gum, collagen, gelatin, cellulose, dextran, dextrin, gum tragacanth, hydroxyethyl cellulose, hydroxypropyl cellulose, sodium hyaluronate pectin, pullulan, methyl cellulose, methyl hydroxypropyl cellulose, and the like. Among, one water-soluble polymer may be used singly, or two or more water-soluble polymers may be used by being mixed together.

In a case where a carboxyvinyl polymer is used to effectively use the silicon oxide-coated zinc oxide of the present invention, the content of the carboxyvinyl polymer in the water-based composition containing silicon oxide-coated zinc oxide of the present invention may be 0.5% by mass or more and 2.5% by mass or less, 1.0% by mass or more and 2.0% by mass or less, or 1.3% by mass or more and 1.7% by mass or less.

The water-soluble polymer functions as a dispersant and a viscosity adjuster. Furthermore, by being added to the water-based composition, the water-soluble polymer functions to improve the dispersibility and the temporal stability of the silicon oxide-coated zinc oxide in the water-based composition containing silicon oxide-coated zinc oxide.

In a case where the water-based composition containing silicon oxide-coated zinc oxide contains a water-soluble polymer, the content rate of alcohols is preferably 5% by mass or more and 20% by mass or less, and more preferably 15% by mass or more and 20% by mass or less. The water-based composition may not contain alcohols.

As described above, in a case where the water-based composition containing silicon oxide-coated zinc oxide contains a water-soluble polymer, the content rate of alcohols is 5% by mass or more and 20% by mass or less, for the following reason. That is, in a case where the content rate of alcohols is less than 5% by mass, depending on the type of the water-soluble polymer, because the content of alcohols is too small, the alcohols fail to evenly infiltrate into the water-soluble polymer, and the water-soluble polymer unevenly swells due to moisture. As a result, it is difficult to handle the composition due to the deterioration of dispersibility of the silicon oxide-coated zinc oxide, and the temporal stability of the water-based composition containing silicon oxide-coated zinc oxide deteriorates. Therefore, it is not preferable that the content rate of alcohols is less than 5% by mass. Furthermore, in a case where the content rate of alcohols is more than 20% by mass, the overall viscosity of the water-based composition containing silicon oxide-coated zinc oxide increases. Consequently, the dispersion stability of the silicon oxide-coated zinc oxide deteriorates, and the temporal stability of the water-based composition containing silicon oxide-coated zinc oxide also deteriorates. Therefore, it is not preferable that the content of alcohols is more than 20% by mass.

The water-based composition containing silicon oxide-coated zinc oxide is obtained by mixing the aforementioned silicon oxide-coated zinc oxide with a water-based dispersion medium, a water-based dispersion medium containing alcohols, a water-based dispersion medium containing a water-soluble polymer, or a water-based dispersion medium containing alcohols and a water-soluble polymer, and then, if necessary, mixing water therewith to disperse the silicon oxide-coated zinc oxide. The amount of water in the composition may be appropriately adjusted. However, considering the dispersion stability and the temporal stability of the silicon oxide-coated zinc oxide, the amount of water is preferably within a range of 15% by mass or more and 94% by mass or less. Within this range, a suitable amount may be selected as necessary.

By adjusting the amount of water within the aforementioned range, a water-based composition containing silicon oxide-coated zinc oxide is obtained which can secure sufficient transparency in a case where the composition is spread and applied onto the skin even if being used singly or mixed with a cosmetic product.

[Cosmetic product]

An example of the cosmetic product of the present embodiment contains the silicon oxide-coated zinc oxide of the present embodiment or the composition containing silicon oxide-coated zinc oxide of the present embodiment.

Another example of the cosmetic product of the present embodiment contains a cosmetic base material and the silicon oxide-coated zinc oxide of the present embodiment or the composition containing silicon oxide-coated zinc oxide of the present embodiment that is dispersed in the cosmetic base material.

The cosmetic base material means various raw materials forming the base of cosmetics, and examples thereof include an oleaginous raw material, an aqueous raw material, a surfactant, a powder raw material, and the like.

Examples of the oleaginous raw material include oil and fat, a higher fatty acid, a higher alcohol, ester oils, and the like.

Examples of the aqueous raw material include purified water, an alcohol, a thickener, and the like.

The powder raw material includes an organic pigment, a white pigment, a pearl agent, an extender pigment, and the like.

The cosmetic product of the present embodiment is obtained by mixing the silicon oxide-coated zinc oxide or composition containing silicon oxide-coated zinc oxide of the present embodiment with a cosmetic base material of an emulsion, a cream, a foundation, a lipstick, a blusher, or an eyeshadow in the manner of the related art.

Furthermore, the silicon oxide-coated zinc oxide or composition containing silicon oxide-coated zinc oxide of the present embodiment may be mixed with an oil phase or a water phase so as to obtain an O/W-type or W/O-type emulsion, and then mixed with the cosmetic base material.

The content of the silicon oxide-coated zinc oxide in the cosmetic product may be appropriately adjusted according to the intended characteristics. For example, the lower limit of the content of the silicon oxide-coated zinc oxide may be 0.01% by mass or more, 0.1% by mass or more, or 1% by mass or more. The upper limit of the content of the silicon oxide-coated zinc oxide may be 50% by mass or less, 40% by mass or less, or 30% by mass or less. The upper limit and the lower limit of the content of the silicon oxide-coated zinc oxide in the cosmetic product can be arbitrarily combined.

In order to effectively use the silicon oxide-coated zinc oxide of the present invention, the cosmetic product of the present invention contains, for example, the silicon oxide-coated zinc oxide and cosmetic base material of the present invention, and the cosmetic base material contains a carboxyvinyl polymer and a water-based solvent. The content rate of the silicon oxide-coated zinc oxide is 0.5% by mass or more and 20% by mass or less, preferably 2% by mass or more and 8% by mass or less, and more preferably 3% by mass or more and 7% by mass or less. The content rate of the carboxyvinyl polymer is 0.1% by mass or more and 10% by mass or less, preferably 0.5% by mass or more and 5% by mass or less, and more preferably 1% by mass or more and 2.5% by mass or less. The pH of the water-based solvent is 6.5 or more and 9 or less, preferably 7 or more and 8.5 or less, and more preferably 7 or more and 8 or less.

Hereinafter, a sunscreen cosmetic product will be specifically described.

In order to effectively shield ultraviolet rays, particularly, long-wavelength ultraviolet rays (UVA), the content rate of the composition containing silicon oxide-coated zinc oxide in the sunscreen cosmetic product is preferably 1% by mass or more and 30% by mass or less, more preferably 3% by mass or more and 20% by mass or less, and even more preferably 5% by mass or more and 15% by mass or less, in terms of the content rate of the silicon oxide-coated zinc oxide contained in the composition containing silicon oxide-coated zinc oxide.

If necessary, the sunscreen cosmetic product may contain a hydrophobic dispersion medium, inorganic fine particles or inorganic pigments other than zinc oxide particles, a hydrophilic dispersion medium, oil and fat, a surfactant, a moisturizer, a thickener, a pH adjuster, a nutrient, an antioxidant, an aromatic, and the like.

Examples of the hydrophobic dispersion medium include hydrocarbon oil such as liquid paraffin, squalane, isoparaffin, branched light paraffin, Vaseline, and ceresin, ester oil such as isoprpyl myristate, cetyl isooctanoate, and glyceryl trioctanoate, silicone oil such as decamethyl cyclopentasiloxane, dimethyl polysiloxane, and methyl phenyl polysiloxane, a higher fatty acid such as lauric acid, myristic acid, palmitic acid, and stearic acid, a higher alcohol such as lauroyl alcohol, cetyl alcohol, stearyl alcohol, hexyl dodecanol, and isostearyl alcohol, and the like.

Examples of the inorganic fine particles or inorganic pigments other than zinc oxide particles include calcium carbonate, calcium phospahte (apatite), magnesium carboante, calcium silicate, magnesium silicate, aluminum silicate, kaolin, talc, titanium oxide, aluminum oxide, yellow iron oxide, $\gamma$-iron oxide, cobalt titanate, cobalt violet, silicon oxide, and the like.

The sunscreen cosmetic product may further contain at least one organic ultraviolet absorber.

Examples of the organic ultraviolet absorber include a benzotriazole-based ultraviolet absorber, a benzoylmethane-based ultraviolet absorber, a benzoic acid-based ultraviolet absorber, an anthranilic acid-based ultraviolet absorber, a salicylic acid-based ultraviolet absorber, a cinnamic acid-based ultraviolet absorber, a silicone-based cinnamic acid ultraviolet absorber, organic ultraviolet absorbers other than these, and the like.

Examples of the benzotriazole-based ultraviolet absorber include 2,2'-hydroxy-5-methylphenylbenzotriazole, 2-(2'-hydroxy-5'-t-octylphenyl)benzotriazole, 2-(2'-hydroxy-5'-methylphenyl)benzotriazole, and the like.

Examples of the benzoylmethane-based ultraviolet absorber include dibenzalazine, dianisoylmethane, 4-tert-butyl-4'-methoxydibenzoylmethane, 1-(4'-isopropylphenyl)-3-phenylpropane-1,3-dione, 5-(3,3'-dimethyl-2-norbornylidene)-3-pentan-2-one, and the like.

Examples of the benzoic acid-based ultraviolet absorber include p-aminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA methyl ester, and the like.

Examples of the anthranilic acid-based ultraviolet absorber include homomenthyl-N-acetyl anthranilate, and the like.

Examples of the salicylic acid-based ultraviolet absorber include amyl salicylate, menthyl salicylate, homomenthyl salicylate, octyl salicylate, phenyl salicylate, benzyl salicylate, p-2-propanol phenyl salicylate, and the like.

Examples of the cinnamic acid-based ultraviolet absorber include octyl methoxycinnamate, glyceryl di-paramethoxy cinnamic acid-mono-2-ethyl hexanoate, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-$\alpha$-cyano-$\beta$-phenyl cinnamate, 2-ethylhexyl-$\alpha$-cyano-$\beta$-phenyl cinnamate, glyceryl mono-2-ethylhexanoyl-diparamethoxycinnamate, and the like.

Examples of the silicone-based cinnamic acid ultraviolet absorber include
[3-bis(trimethylsiloxy)methylsilyl-1-methylpropyl]-3,4,5-tr imethoxycinnamate,
[3-bis(trimethylsiloxy)methylsilyl-3-methylpropyl]-3,4,5-tr imethoxycinnamate,
[3-bis(trimethylsiloxy)methylsilylpropyl]-3,4,5-trimethoxyc innamate,
[3-bis(trimethylsiloxy)methylsilylbutyl]-3,4,5-trimethoxyci nnamate,
[3-tris(trimethylsiloxy)silylbutyl]-3,4,5-trimethoxycinnama te,

[3-tris(trimethylsiloxy)silyl-1-methylpropyl]-3,4-dimethoxy cinnamate, and the like.

Examples of organic ultraviolet absorbers other than the above include 3-(4'-methylbenzylidene)-d,1-camphor, 3-benzylidene-d,1-camphor, urocanic acid, urocanic acid ethyl ester, 2-phenyl-5-methyl benzoxazole, 5-(3,3'-dimethyl-2-norbornylidene)-3-pentan-2-one, a silicone-modified ultraviolet absorber, a fluorine-modified ultraviolet absorber, and the like.

As described above, the electric conductivity of a water suspension of the silicon oxide-coated zinc oxide of the present embodiment is 120 $\mu$S/cm or less. Therefore, even in a case where the silicon oxide-coated zinc oxide is used in water-based materials such as water-based cosmetic products, the decrease in viscosity resulting from a carbomer can be inhibited, and hence the quality stability of the water-based materials can be maintained.

The composition containing silicon oxide-coated zinc oxide of the present embodiment contains the silicon oxide-coated zinc oxide of the present embodiment. Therefore, even in a case where the composition is used in water-based materials such as water-based cosmetic products, the decrease in viscosity resulting from a carbomer can be inhibited, and hence the quality stability of the water-based materials can be maintained.

The cosmetic product of the present embodiment contains the composition containing silicon oxide-coated zinc oxide of the present embodiment. Therefore, the decrease in viscosity resulting from a carbomer does not occur in the cosmetic product, and the cosmetic product has excellent quality stability. Examples

Hereinafter, the present invention will be more specifically described based on examples and comparative examples, but the present invention is not limited to the following examples.

[Example 1]

[Silicon oxide-coated zinc oxide]

Zinc oxide particles (average particle diameter: 35 nm; manufactured by SUMITOMO OSAKA CEMENT Co., Ltd.) were mixed with water and then dispersed using ultrasonic waves, thereby preparing a water-based zinc oxide suspension in which the content rate of the zinc oxide particles was 20% by mass.

Thereafter, the water-based zinc oxide suspension was added to an aqueous sodium silicate solution which contained sodium silicate in an amount of 20% by mass in terms of silicon oxide with respect to the mass of the zinc oxide particles in the water-based zinc oxide suspension, followed by stirring, thereby obtaining a suspension.

Then, the suspension was heated to 60°C, and dilute sulfuric acid was slowly added to the suspension with stirring such that the pH was adjusted and became 6.5 to 7. Subsequently, the suspension was left to stand for 2 hours, an aqueous calcium chloride solution (25% by mass of calcium chloride dihydrate) having the same mass as the zinc oxide particles in the suspension was added thereto, followed by stirring, and then the suspension was left to stand for 2 more hours.

Thereafter, the suspension was subjected to solid-liquid separation by using a centrifuge, and the obtained solid was cleaned with water by using a filter press. Then, the solid was dried at 150°C and subjected to a thermal treatment (calcination) for 1 hour at 500°C.

Then, the obtained solid was mixed with 2-propanol and then dispersed using ultrasonic waves, thereby preparing a 2-propanol suspension of silicon oxide-coated zinc oxide in which the content rate of the silicon oxide-coated zinc oxide was 10% by mass.

Thereafter, the suspension was heated to 60°C, and aqueous ammonia and water were added to the suspension with stirring such that the pH was adjusted and became 10 to 11. The amount of water added was 120% by mass with respect to tetraethoxysilane in a tetraethoxysilane 2-propanol solution which will be added later.

A tetraethoxysilane 2-propanol solution was slowly added dropwise to the suspension such that the amount of tetraethoxysilane added dropwise became 15% by mass with respect to the total mass of zinc oxide in terms of silicon oxide, and the suspension was kept stirred for 6 hours.

After the reaction ended, the suspension was subjected to solid-liquid separation by using a centrifuge, and the obtained solid was dried at 150°C. Then, a thermal treatment (calcination) was performed on the dried substance for 3 hours at 500°C, thereby preparing silicon oxide-coated zinc oxide of Example 1.

By atomic absorption spectroscopy, the contents of Na and Ca in the silicon oxide-coated zinc oxide of Example 1 were measured. As a result, the contents of Na and Ca were 0.20% by mass and 0.32% by mass respectively.

Furthermore, the NMR spectrum of the silicon oxide-coated zinc oxide of Example 1 was measured by MAS-nuclear magnetic resonance (NMR) spectroscopy by using solid-state $^{29}$Si. From the peak area ratio of the NMR spectrum, an area ratio $Q^0$, $Q^1$, $Q^2$, $Q^3$, or $Q^4$ of signals attributed to each of $Q^0$, $Q^1$, $Q^2$, $Q^3$, and $Q^4$ environments was calculated.

For silicon in the silicon oxide coating constituting the silicon oxide-coated zinc oxide of Example 1, an abundance ratio of the silicon in the $Q^3$ environment was denoted by $Q^3$, and an abundance ratio thereof in the $Q^4$ environment was denoted by $Q^4$. Then, a value of $Q^3 + Q^4$ and a value of $Q^4/(Q^3 + Q^4)$ were calculated. As a result, $Q^3 + Q^4 \geq 0.6$, and $Q^4/(Q^3 + Q^4) \geq 0.5$.

[Composition containing silicon oxide-coated zinc oxide]

A carbomer (trade name: Ultrez 10, manufactured by Nikko Chemicals Co., Ltd.) was dissolved in 1.5 g of pure water. Then, 10% by mass of an aqueous sodium hydroxide solution was added dropwise thereto so as to adjust the pH, thereby preparing an aqueous carbomer solution with pH of 7.5 that contained 1.5% by mass of a carbomer.

Thereafter, the aqueous carbomer solution was mixed with the silicon oxide-coated zinc oxide of Example 1 at a mass ratio of 95:5, followed by stirring, thereby obtaining a composition containing silicon oxide-coated zinc oxide of Example 1.

[Example 2]

[Silicon oxide-coated zinc oxide]

Zinc oxide particles (average particle diameter: 35 nm; manufactured by SUMITOMO OSAKA CEMENT Co., Ltd.) was mixed with water and then dispersed using ultrasonic waves, thereby preparing a water-based zinc oxide suspension in which the content rate of zinc oxide particles was 20% by mass.

Then, the water-based zinc oxide suspension was added to an aqueous sodium silicate solution which contained sodium silicate in an amount of 20% by mass in terms of silicon oxide with respect to the mass of the zinc oxide particles in the water-based zinc oxide suspension, followed by stirring, thereby obtaining a suspension.

Then, the suspension was heated to 60°C, and dilute sulfuric acid was slowly added to the suspension with stirring such that the pH was adjusted and became 6.5 to 7. Subsequently, the suspension was left to stand for 2 hours and then subjected to solid-liquid separation by using a centrifuge, and the obtained solid was cleaned with water by using a filter press.

Thereafter, an aqueous calcium chloride solution (25% by mass of calcium chloride dihydrate) having the same mass as the zinc oxide particles in the solid was added thereto, and then the suspension was left to stand for 2 more hours. Subsequently, the suspension was subjected to solid-liquid separation by using a centrifuge, and the obtained solid was cleaned again with water by using a filter press. Then, the solid was dried at 150°C and subjected to a thermal treatment (calcination) for 1 hour at 500°C.

Then, the obtained solid was mixed with 2-propanol and then dispersed using ultrasonic waves, thereby preparing a 2-propanol suspension of silicon oxide-coated zinc oxide in which the content rate of the silicon oxide-coated zinc oxide was 10% by mass.

Thereafter, the suspension was heated to 60°C, and aqueous ammonia and water were added to the suspension with stirring such that the pH was adjusted and became 10 to 11. The amount of water added was 120% by mass with respect to tetraethoxysilane in a tetraethoxysilane 2-propanol solution which will be added later.

A tetraethoxysilane 2-propanol solution was slowly added dropwise to the suspension such that the amount of tetraethoxysilane added dropwise became 15% by mass with respect to the total mass of zinc oxide in terms of silicon oxide, and the suspension was kept stirred for 6 hours.

After the reaction ended, the suspension was subjected to solid-liquid separation by using a centrifuge, and the obtained solid was dried at 150°C. Then, a thermal treatment (calcination) was performed on the dried substance for 3 hours at 500°C, thereby preparing silicon oxide-coated zinc oxide of Example 2.

By atomic absorption spectroscopy, the contents of Na and Ca in the silicon oxide-coated zinc oxide of Example 2 were measured. As a result, the contents of Na and Ca were 0.10% by mass and 0.17% by mass respectively.

Furthermore, in the same manner as in Example 1, for silicon in the silicon oxide coating constituting the silicon oxide-coated zinc oxide of Example 2, an abundance ratio of the silicon in the $Q^3$ environment was denoted by $Q^3$, and an abundance ratio thereof in the $Q^4$ environment was denoted by $Q^4$. Then, a value of $Q^3 + Q^4$ and a value of $Q^4/(Q^3 + Q^4)$ were calculated. As a result, $Q^3 + Q^4 \geq 0.6$, and $Q^4/(Q^3 + Q^4) \geq 0.5$.

[Composition containing silicon oxide-coated zinc oxide]

A composition containing silicon oxide-coated zinc oxide of Example 2 was obtained in the same manner as in Example 1, except that the silicon oxide-coated zinc oxide of Example 2 was used instead of the silicon oxide-coated zinc oxide of Example 1.

[Example 3]

[Silicon oxide-coated zinc oxide]

Zinc oxide particles (average particle diameter: 250 nm; manufactured by SUMITOMO OSAKA CEMENT Co.,

Ltd.) were mixed with water and then dispersed using ultrasonic waves, thereby preparing a water-based zinc oxide suspension in which the content rate of the zinc oxide particles was 50% by mass.

Thereafter, the water-based zinc oxide suspension was added to an aqueous sodium silicate solution which contained sodium silicate in an amount of 17.7% by mass in terms of silicon oxide (content of zinc oxide in the silicon oxide-coated zinc oxide is 15% by mass) with respect to the mass of the zinc oxide particles in the water-based zinc oxide suspension, followed by stirring, thereby obtaining a suspension.

Then, the suspension was heated to 60°C, and dilute sulfuric acid was slowly added to the suspension with stirring such that the pH was adjusted and became 6. Subsequently, the suspension was left to stand for 2 hours, an aqueous calcium chloride solution (50% by mass of calcium chloride dihydrate) having the same mass as the zinc oxide particles in the suspension was added thereto, and then the suspension was left to stand for 2 more hours.

Thereafter, the suspension was subjected to solid-liquid separation by using a centrifuge, and the obtained solid was suspended in water and stirred for 10 minutes, and then solid-liquid separation was performed using a centrifuge. The step of suspending and cleaning was performed 5 times in total.

Then, the solid was dried at 150°C and subjected to a thermal treatment (calcination) for 3 hours at 500°C, thereby preparing silicon oxide-coated zinc oxide of Example 3.

By atomic absorption spectroscopy, the contents of Na and Ca in the silicon oxide-coated zinc oxide of Example 3 were measured. As a result, the contents of Na and Ca were 0.12% by mass and 0.10% by mass respectively.

Furthermore, in the same manner as in Example 1, for silicon in the silicon oxide coating constituting the silicon oxide-coated zinc oxide of Example 3, an abundance ratio of the silicon in the $Q^3$ environment was denoted by $Q^3$, and an abundance ratio thereof in the $Q^4$ environment was denoted by $Q^4$. Then, a value of $Q^3 + Q^4$ and a value of $Q^4/(Q^3 + Q^4)$ were calculated. As a result, $Q^3 + Q^4 \geq 0.6$, and $Q^4/(Q^3 + Q^4) < 0.5$.

[Composition containing silicon oxide-coated zinc oxide]

A composition containing silicon oxide-coated zinc oxide of Example 3 was obtained in the same manner as in Example 1, except that the silicon oxide-coated zinc oxide of Example 3 was used instead of the silicon oxide-coated zinc oxide of Example 1.

[Example 4]

[Silicon oxide-coated zinc oxide]

Silicon oxide-coated zinc oxide of Example 4 was prepared in the same manner as in Example 3, except that the step of suspending the solid in water and cleaning the solid in Example 3 was performed 8 times in total.

By atomic absorption spectroscopy, the contents of Na and Ca in the silicon oxide-coated zinc oxide of Example 4 were measured. As a result, the contents of Na and Ca were 0.11% by mass and 0.13% by mass respectively.

Furthermore, in the same manner as in Example 1, for silicon in the silicon oxide coating constituting the silicon oxide-coated zinc oxide of Example 4, an abundance ratio of the silicon in the $Q^3$ environment was denoted by $Q^3$, and an abundance ratio thereof in the $Q^4$ environment was denoted by $Q^4$. Then, a value of $Q^3 + Q^4$ and a value of $Q^4/(Q^3 + Q^4)$ were calculated. As a result, $Q^3 + Q^4 \geq 0.6$, and $Q^4/(Q^3 + Q^4) < 0.5$.

[Composition containing silicon oxide-coated zinc oxide]

A composition containing silicon oxide-coated zinc oxide of Example 4 was obtained in the same manner as in Example 1, except that the silicon oxide-coated zinc oxide of Example 4 was used instead of the silicon oxide-coated zinc oxide of Example 1.

[Comparative Example 1]

[Silicon oxide-coated zinc oxide]

Silicon oxide-coated zinc oxide of Comparative Example 1 was prepared in the same manner as in Example 3, except that the step of suspending the solid in water and cleaning the solid in Example 3 was performed 4 times in total.

In the same manner as in Example 1, for silicon in the silicon oxide coating constituting the silicon oxide-coated zinc oxide of Comparative Example 1, an abundance ratio of the silicon in the $Q^3$ environment was denoted by $Q^3$, and an abundance ratio thereof in the $Q^4$ environment was denoted by $Q^4$. Then, a value of $Q^3 + Q^4$ and a value of $Q^4/(Q^3 + Q^4)$ were calculated. As a result, $Q^3 + Q^4 \geq 0.6$, and $Q^4/(Q^3 + Q^4) < 0.5$.

[Composition containing silicon oxide-coated zinc oxide]

A composition containing silicon oxide-coated zinc oxide of Comparative Example 1 was obtained in the same manner as in Example 1, except that the silicon oxide-coated zinc oxide of Comparative Example 1 was used instead of the silicon oxide-coated zinc oxide of Example 1.

[Comparative Example 2]

[Silicon oxide-coated zinc oxide]

Zinc oxide particles (average particle diameter: 35 nm; manufactured by SUMITOMO OSAKA CEMENT Co., Ltd.) were mixed with water and then dispersed using ultrasonic waves, thereby preparing a water-based zinc oxide suspension in which the content rate of the zinc oxide particles was 50 g/L (5% by mass) .

Thereafter, the suspension was heated to 80°C, an aqueous sodium silicate solution was added to the suspension with stirring such that the suspension contained sodium silicate in an amount of 10% by mass in terms of silicon oxide with respect to the mass of the zinc oxide particles in the water-based zinc oxide suspension, and the suspension was matured for 10 minutes.

Then, dilute sulfuric acid was added for 60 minutes to the suspension with stirring such that the pH was adjusted and became 6.5, and the suspension was matured for 30 minutes.

Subsequently, an aqueous sodium aluminate solution was added to the suspension with stirring such that the amount of sodium aluminate became 5% by mass in terms of aluminum oxide with respect to the total mass of the zinc oxide particles, and the suspension was matured for 10 minutes.

Thereafter, dilute sulfuric acid was added for 10 minutes to the suspension with stirring such that the pH was adjusted and became 7.0, and the suspension was matured for 30 minutes.

Then, the suspension was subjected to solid-liquid separation by using a centrifuge and cleaned with water, and the obtained solid was heated and dried for 5 hours at 130°C.

The dried substance was then crushed using a jet mill, thereby preparing silicon oxide-coated zinc oxide of Comparative Example 2.

[Composition containing silicon oxide-coated zinc oxide]

A composition containing silicon oxide-coated zinc oxide of Comparative Example 2 was obtained in the same manner as in Example 1, except that the silicon oxide-coated zinc oxide of Comparative Example 2 was used instead of the silicon oxide-coated zinc oxide of Example 1.

[Comparative Example 3]

[Silicon oxide-coated zinc oxide]

As silicon oxide-coated zinc oxide of Comparative Example 3, commercially available silicon oxide-coated zinc oxide (trade name: SiH20-ZnO650, average particle diameter: 25 nm, $SiO_2$/ZnO = 17% by mass, manufactured by SUMITOMO OSAKA CEMENT Co., Ltd.) was used.

[Composition containing silicon oxide-coated zinc oxide]

A composition containing silicon oxide-coated zinc oxide of Comparative Example 3 was obtained in the same manner as in Example 1, except that the silicon oxide-coated zinc oxide of Comparative Example 3 was used instead of the silicon oxide-coated zinc oxide of Example 1.

[Comparative Example 4]

[Silicon oxide-coated zinc oxide]

As silicon oxide-coated zinc oxide of Comparative Example 4, commercially available silicon oxide-coated zinc oxide (trade name: SiH5-ZnO650, average particle diameter: 25 nm, $SiO_2$/ZnO = 4.77% by mass, manufactured by SUMITOMO OSAKA CEMENT Co., Ltd.) was used.

[Composition containing silicon oxide-coated zinc oxide]

A composition containing silicon oxide-coated zinc oxide of Comparative Example 4 was obtained in the same manner as in Example 1, except that the silicon oxide-coated zinc oxide of Comparative Example 4 was used instead of the silicon oxide-coated zinc oxide of Example 1.

[Evaluation]

[Evaluation of electric conductivity of water suspension of silicon oxide-coated zinc oxide]

10 g of silicon oxide-coated zinc oxide of Examples 1 to 4 and Comparative Examples 1 to 4 was added to 90 g of pure water, boiled for 10 minutes with stirring, and left to cool to 25°C. Then, pure water in the same amount as the amount of water having evaporated was added thereto, thereby preparing a water suspension containing silicon oxide-coated zinc oxide in an amount of 10% by mass.

The electric conductivity of the water suspension was measured using a conductivity meter (trade name: PERSONAL SC METER SC 72, manufactured by Yokogawa Electric Corporation). The results are shown in Table 1.

[Evaluation of viscosity of composition containing silicon oxide-coated zinc oxide]

The viscosity of the composition containing silicon oxide-coated zinc oxide of Examples 1 to 4 and Comparative Examples 1 to 4 was measured using BII-type rotary viscometer (manufactured by TOKI SANGYO CO., LTD.) under the condition of 20°C and 30 rpm.

Furthermore, a predetermined amount of sample was collected from the composition containing silicon oxide-coated zinc oxide of Examples 1 to 4 and Comparative Examples 1 to 4. The collected sample was kept at 40°C in a thermostatic bath, and the viscosity thereof was measured at a predetermined time interval under the condition of 20°C and 30 rpm. The results are shown in Table 1. In a case where a composition containing silicon oxide-coated zinc oxide maintained a viscosity of 5 Pa·s or more for 300 hours from when the sample started to be kept at 40°C, the composition was evaluated to be "GOOD". In a case where a composition containing silicon oxide-coated zinc oxide maintained a viscosity of less than 5 Pa·s for 300 hours from when the sample started to be kept at 40°C, the composition was evaluated to be "NOT GOOD". The results are shown in Table 1. The viscosity of the compositions containing silicon oxide-coated zinc oxide of Comparative Examples 2, 3, and 4 was reduced and became 3 Pa·s or less immediately after mixing. Therefore, these compositions were not subjected to the test of keeping them at 40°C and measuring the viscosity at a predetermined time interval.

The sample of Example 1 was kept at 40°C for 780 hours in the thermostatic bath, and then the pH was measured at 20°C. As a result, the pH was 8.9.

Furthermore, the sample of Example 3 was kept at 40°C for 780 hours in the thermostatic bath, and then the pH was measured at 20°C. As a result, the pH was 9.2.

[Table 1]

|  | Electric conductivity (μS/cm) | Determination |
|---|---|---|
| Example 1 | 99.7 | GOOD |
| Example 2 | 51.1 | GOOD |
| Example 3 | 87.9 | GOOD |
| Example 4 | 45.9 | GOOD |
| Comparative Example 1 | 185 | NOT GOOD |
| Comparative Example 2 | 345 | NOT GOOD |
| Comparative Example 3 | 535 | NOT GOOD |
| Comparative Example 4 | 327 | NOT GOOD |

From the results shown in Table 1, it was understood that the electric conductivity of the water suspension of the silicon oxide-coated zinc oxide of Examples 1 to 4 is 100 μS/cm or less.

In contrast, it was understood that the electric conductivity of the water suspension of the silicon oxide-coated zinc oxide of Comparative Examples 1 to 4 is 185 μS/cm or more.

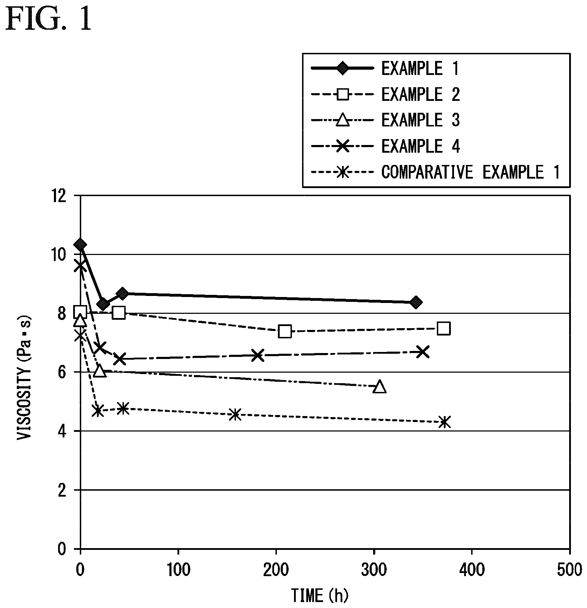

From the results shown in Table 1 and FIG. 1, it was understood that the compositions containing silicon oxide-coated zinc oxide of Examples 1 to 4 maintain a viscosity of 5 Pa·s or more for 300 hours from when the compositions started to be kept at 40°C.

In contrast, it was understood that that the compositions containing silicon oxide-coated zinc oxide of Comparative Examples 1 to 4 maintain a viscosity of less than 5 Pa·s for 300 hours from when the compositions started to be kept at 40°C.

[Observation of silicon oxide-coated zinc oxide]

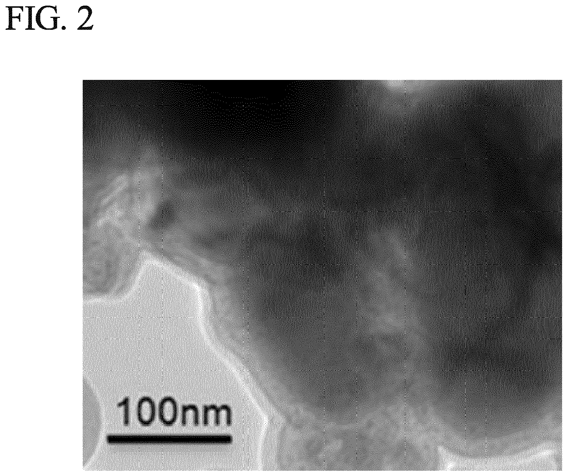

The silicon oxide-coated zinc oxide of Example 3 was observed using an ionizing radiation-type electron microscope (FE-TEM) JEM-2100F (manufactured by JEOL Ltd). The observed micrograph is shown in FIG. 2.

From FIG. 2, it was confirmed that each of the most of the zinc oxide particles is evenly coated with silicon oxide coating, and that the homogeneity of the silicon oxide coating is high.

Industrial Applicability

By setting the electric conductivity of a water suspension of the silicon oxide-coated zinc oxide of the present invention to be 120 μS/cm or less, even in a case where the silicon oxide-coated zinc oxide is used in water-based materials such as water-based cosmetic products, a decrease in viscosity resulting from a carbomer can be inhibited. Accordingly, the silicon oxide-coated zinc oxide of the present invention enables water-based materials to maintain quality stability. Therefore, in a case where the silicon oxide-coated zinc oxide is used in water-based cosmetic products for which a change in a hydrogen ion exponent (pH) of water-based materials needs to be inhibited, a degree of freedom of formulation can be improved. Consequently, the silicon oxide-coated zinc oxide is industrially extremely valuable.

**Claims**

1.  Silicon oxide-coated zinc oxide comprising:

    a zinc oxide particle; and
    a silicon oxide coating on a surface of the zinc oxide particle,
    wherein:

    an electric conductivity of a water suspension of the silicon oxide-coated zinc oxide is 120 μS/cm or less;
    an average particle diameter of the zinc oxide particles is 50 nm or more and 500 nm or less;
    the silicon oxide coating comprises at least one element selected from the group consisting of Mg, Ca, and Ba;
    a total mass percentage of Mg, Ca, and Ba is larger than a total mass percentage of alkali metals;
    the total mass percentage of alkali metals is 0.0001% by mass or more and 0.2% by mass or less; and
    the total mass percentage of Mg, Ca, and Ba is 0.01% by mass or more and 1% by mass or less.

2.  A composition containing silicon oxide-coated zinc oxide comprising:
    the silicon oxide-coated zinc oxide according to claim 1.

3.  The composition containing silicon oxide-coated zinc oxide according to claim 2, further comprising:

    a carboxyvinyl polymer,
    wherein the composition has a viscosity of 5 Pa·s or more.

4.  A cosmetic product comprising:
    the composition containing silicon oxide-coated zinc oxide according to claim 2 or 3.

5.  A cosmetic product comprising:

    the silicon oxide-coated zinc oxide according to claim 1; and
    a cosmetic base material.

**Patentansprüche**

1. Siliziumoxidbeschichtetes Zinkoxid, das enthält:

   ein Zinkoxidteilchen; und
   eine Siliziumoxidbeschichtung auf einer Oberfläche des Zinkoxidteilchens, wobei:

   die elektrische Leitfähigkeit einer wässrigen Suspension des siliziumoxidbeschichteten Zinkoxids 120 $\mu$S/cm oder weniger beträgt;
   der durchschnittliche Teilchendurchmesser der Zinkoxidteilchen 50 nm oder mehr und 500 nm oder weniger beträgt;
   die Siliziumoxidbeschichtung mindestens ein Element enthält, das aus der Gruppe bestehend aus Mg, Ca, und Ba ausgewählt wird;
   der Gesamtmassenanteil von Mg, Ca, und Ba größer ist als der Gesamtmassenanteil von Alkalimetallen;
   der Gesamtmassenanteil von Alkalimetallen 0.0001 Masse-% oder mehr und 0 .2% Masse-% oder weniger beträgt; und
   der Gesamtmassenanteil von Mg, Ca, und Ba 0.01 Masse-% oder mehr und 1 Masse-% oder weniger beträgt.

2. Eine Zusammensetzung mit siliziumoxidbeschichteten Zinkoxid, die enthält:
   das siliziumoxidbeschichtete Zinkoxid nach Anspruch 1.

3. Zusammensetzung mit siliziumoxidbeschichtetem Zinkoxid nach Anspruch 2, weiterhin umfassend:

   ein Carboxyvinyl- Polymer,
   wobei die Zusammensetzung eine Viskosität von 5 Pa•s oder mehr hat.

4. Ein kosmetisches Produkt, das enthält:
   die Zusammensetzung mit siliziumoxidbeschichtetem Zinkoxid nach Anspruch 2 oder 3.

5. Ein kosmetisches Produkt, das enthält:

   das siliziumoxidbeschichtete Zinkoxid nach Anspruch 1; und
   einen kosmetischen Grundstoff.

**Revendications**

1. Oxyde de zinc recouvert d'oxyde de silicium, comprenant :

   une particule d'oxyde de zinc ; et
   un revêtement d'oxyde de silicium sur une surface de la particule d'oxyde de zinc,
   dans lequel :

   une conductivité électrique d'une suspension aqueuse de l'oxyde de zinc recouvert d'oxyde de silicium est égale à 120 $\mu$S/cm, ou moins ;
   un diamètre de particule moyen des particules d'oxyde de zinc est égal à 50 nm, ou plus, et à 500 nm, ou moins ;
   le revêtement d'oxyde de silicium comprend au moins un élément qui est sélectionné dans le groupe comprenant le Mg, le Ca et le Ba ;
   un pourcentage en masse de Mg, de Ca et de Ba est supérieur à un pourcentage en masse total de métaux alcalins ;
   le pourcentage en masse total des métaux alcalins est égal à 0,0001 % en masse, ou plus, et à 0,2 % en masse, ou moins ; et
   le pourcentage en masse total de Mg, de Ca et de Ba est égal à 0,01 % en masse, ou plus, et à 1 % en masse, ou moins

2. Composition contenant de l'oxyde de zinc recouvert d'oxyde de silicium, comprenant :

l'oxyde de zinc recouvert d'oxyde de silicium selon la revendication 1.

3. Composition contenant de l'oxyde de zinc recouvert d'oxyde de silicium selon la revendication 2, comprenant en outre :

   un polymère carboxyvinylique, et
   dans laquelle la composition présente une viscosité égale à 5 Pa·s, ou plus.

4. Produit cosmétique, comprenant :
   la composition contenant de l'oxyde de zinc recouvert d'oxyde de silicium selon la revendication 2 ou 3.

5. Produit cosmétique, comprenant :

   l'oxyde de zinc recouvert d'oxyde de silicium selon la revendication 1 ; et
   une matière cosmétique de base.

FIG. 1

FIG. 2

**EP 3 263 525 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015038674 A
- JP 3183620 A
- JP 11256133 A
- JP 11302015 A
- JP 2007016111 A
- WO 2014171322 A